(19) Europäisches Patentamt
European Patent Office
Office européen des brevets

(11) Veröffentlichungsnummer: **0 291 797 A2**

# (12) EUROPÄISCHE PATENTANMELDUNG

(21) Anmeldenummer: **88107380.3**

(22) Anmeldetag: **07.05.88**

(51) Int. Cl.4: **C07D 249/08 , A01N 43/653 , C07D 333/22 , C07C 43/20 , C07C 25/24**

(30) Priorität: **18.05.87 DE 3716558**

(43) Veröffentlichungstag der Anmeldung: **23.11.88 Patentblatt 88/47**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Holmwood, Graham, Dr.**
**Krutscheider Weg 105**
**D-5600 Wuppertal 11(DE)**
Erfinder: **Jäger, Gerhard, Dr.**
**Paul-Klee-Strasse 68**
**D-5090 Leverkusen(DE)**
Erfinder: **Büchel, Karl Heinz, Prof. Dr.**
**Dabringhausener Strasse 42**
**D-5093 Burscheid(DE)**
Erfinder: **Brandes, Wilhelm, Dr.**
**Eichendorffstrasse 3**
**D-5653 Leichlingen(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**

## (54) Hydroxyalkyl-azolyl-Derivate.

(57) Neue Hydroxyalkyl-azolyl-Derivate der Formel

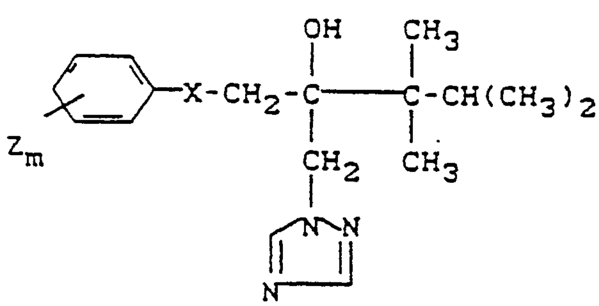

(I)

in welcher
Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenal-

EP 0 291 797 A2

kylthio mit 1 oder 2 Kohlenstoffatomen und 1 bis 5 Halogenatomen, Phenyl oder Alkoximinomethyl mit 1 bis 4 Kohlenstoffatomen in der Alkoxygruppe steht,
X für Sauerstoff, Schwefel oder eine $CH_2$-Gruppe steht und
m für die Zahlen 0, 1, 2 oder 3 steht,
sowie deren Säureadditions-Salze und Metallsalz-Komplexe,
ein Verfahren zur Herstellung der neuen Stoffe und deren Verwendung als Fungizide und Pflanzenwachstumsregulatoren.

Neue Zwischenprodukte und deren Verwendung zur Herstellung von Hydroxyalkyl-azolyl-Derivaten der Formel (I).

## Hydroxyalkyl-azolyl-Derivate

Die vorliegende Erfindung betrifft neue Hydroxyalkyl-azolyl-Derivate, ein Verfahren zu ihrer Herstellung sowie ihre Verwendung als Fungizide und Pflanzenwachstumsregulatoren.

Es ist bereits bekannt, daß zahlreiche Hydroxyalkyl-azolyl-Derivate fungizide und pflanzenwachstumsregulierende Eigenschaften besitzen (vgl. EP-OS 0 040 345 und EP-OS 0 061 835). So lassen sich z.B. 2-(4-(Chlorphenoxy-methyl)-3,3-dimethyl-1-(1,2,4-triazol-1-yl)-butan-2-ol und 1-(4-Chlorphenyl)-4,4-dimethyl-3-(1,2,4-triazol-1-yl-methyl)-pentan-3-ol zur Bekämpfung von Pilzen und zur Regulierung des Pflanzenwachstums einsetzen. Die Wirksamkeit dieser Stoffe ist im allgemeinen sehr gut; allerdings läßt die Pflanzenverträglichkeit und auch die Wirksamkeit in manchen Fällen zu wünschen übrig.

Es wurden nun neue Hydroxyalkyl-azolyl-Derivate der Formel

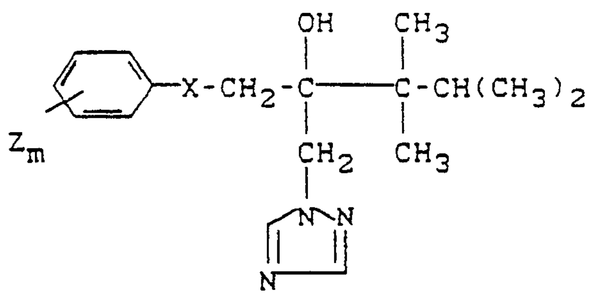

(I)

in welcher

Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl oder Alkoximinomethyl mit 1 bis 4 Kohlenstoffatomen in der Alkoxygruppe steht,

X für Sauerstoff, Schwefel oder eine $CH_2$-Gruppe steht und

m für die Zahlen 0, 1, 2 oder 3 steht,

sowie deren Säureadditions-Salze und Metallsalz-Komplexe gefunden.

Die neuen Hydroxyalkyl-azolyl-Derivate der Formel (I) besitzen ein asymmetrisch substituiertes Kohlenstoffatom und können deshalb in den beiden optischen Isomerenformen anfallen. Die Erfindung betrifft sowohl die Racemate als auch die einzelnen Isomeren und deren Gemische.

Weiterhin wurde gefunden, daß man Hydroxyalkyl-azolyl-Derivate der Formel (I) sowie deren Säureadditions-Salze und Metallsalz-Komplexe erhält, wenn man Oxirane der Formel

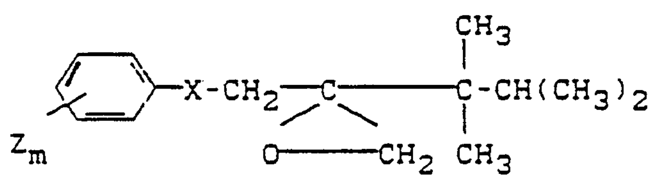

(II)

in welcher

X, Z und m die oben angegebene Bedeutung haben,

mit 1,2,4-Triazol der Formel

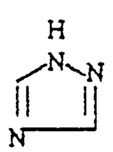

(III)

in Gegenwart eines Verdünnungsmittels und gegebenenfalls in Gegenwart eines Säurebindemittels sowie gegebenenfalls in Gegenwart eines Katalysators umsetzt und gegebenenfalls anschließend an die so erhaltenen Verbindungen der Formel (I) eine Säure oder ein Metallsalz addiert.

Schließlich wurde gefunden, daß sich die Hydroxyalkyl-azolyl-Derivate der Formel (I) sowie deren Säureadditions-Salze und Metallsalz-Komplexe durch sehr gute fungizide und pflanzenwachstumsregulierende Eigenschaften auszeichnen.

Überraschenderweise besitzen die erfindungsgemäßen Stoffe eine bessere fungizide und pflanzenwachstumsregulierende Wirksamkeit als 1-(4-Chlorphenoxy-methyl)-3,3-dimethyl-1-(1,2,4-triazol-1-yl)-butan-2-ol und 1-(4-Chlorphenyl)-4,4-dimethyl-3-(1,2,4-triazol-1-yl-methyl)-pentan-3-ol, welches konstitutionell ähnliche, vorbekannte Wirkstoffe gleicher Wirkungsrichtung sind. Außerdem weisen die erfindungsgemäßen Stoffe eine hervorragende Pflanzenverträglichkeit auf.

Die erfindungsgemäßen Hydroxyalkyl-azolyl-Derivate sind durch die Formel (I) allgemein definiert. Bevorzugt sind diejenigen Verbindungen der Formel (I), in denen

X für Sauerstoff, Schwefel oder eine $CH_2$-Gruppe steht,

Z für Fluor, Chlor, Brom, Methyl, Ethyl, Trifluormethyl, Trifluormethoxy, Trifluormethylthio, Phenyl, Methoximinomethyl oder Ethoximinomethyl steht und

m für die Zahlen 0, 1, 2 oder 3 steht.

Wenn m für 2 oder 3 steht, können die Substituenten für Z gleich oder verschieden sein.

Bevorzugte erfindungsgemäße Verbindungen sind auch Additionsprodukte aus Säuren und denjenigen Hydroxyalkyl-azolyl-Derivaten der Formel (I), in denen X, Z und m diejenigen Bedeutungen haben, die bereits im Zusammenhang mit der Beschreibung der erfindungsgemäßen Stoffe für diese Substituenten bzw. diesen Index als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Hydroxyalkyl-azolyl-Derivaten der Formel (I), in denen X, Z und m diejenigen Bedeutungen haben, die bereits im Zusammenhang mit der Beschreibung der erfindungsgemäßen Stoffe für diese Substituenten bzw. diesen Index als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzen-physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Verbindungen seien die in der folgenden Tabelle aufgeführten Stoffe genannt.

Tabelle

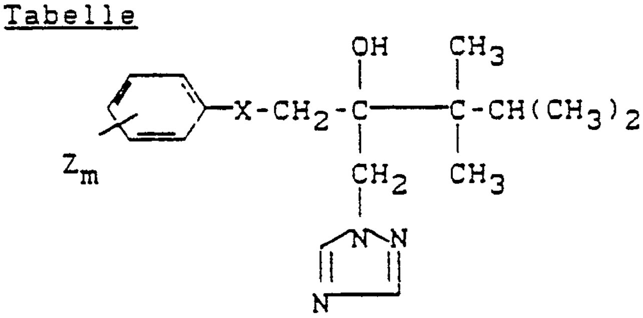

(I)

| X | $Z_m$ |
|---|---|
| O | 4-F |
| S | 4-F |
| $CH_2$ | 4-F |
| O | 2,4-$Cl_2$ |
| S | 2,4-$Cl_2$ |
| $CH_2$ | 2,4-$Cl_2$ |
| O | 4-$CH_3$ |
| S | 4-$CH_3$ |
| $CH_2$ | 4-$CH_3$ |
| O | 4-$CF_3$ |
| S | 4-$CF_3$ |
| $CH_2$ | 4-$CF_3$ |
| O | 4-CH=N-$OCH_3$ |
| S | 4-CH=N-$OCH_3$ |
| $CH_2$ | 4-CH=N-$OCH_3$ |
| O | 4-$OCF_3$ |
| S | 4-$OCF_3$ |
| $CH_2$ | 4-$OCF_3$ |
| $CH_2$ | - |
| O | 4-CH=$NOC_2H_5$ |
| S | 4-CH=$NOC_2H_5$ |
| $CH_2$ | 4-CH=$NOC_2H_5$ |

Verwendet man 2-[2-(4-Chlorphenyl)-ethyl]-2-[(1,1,2-trimethyl)-propyl]-oxiran und 1,2,4-Triazol als Ausgangssubstanzen, so kann der Verlauf des erfindungsgemäßen Verfahrens durch das folgende Formelschema wiedergegeben werden:

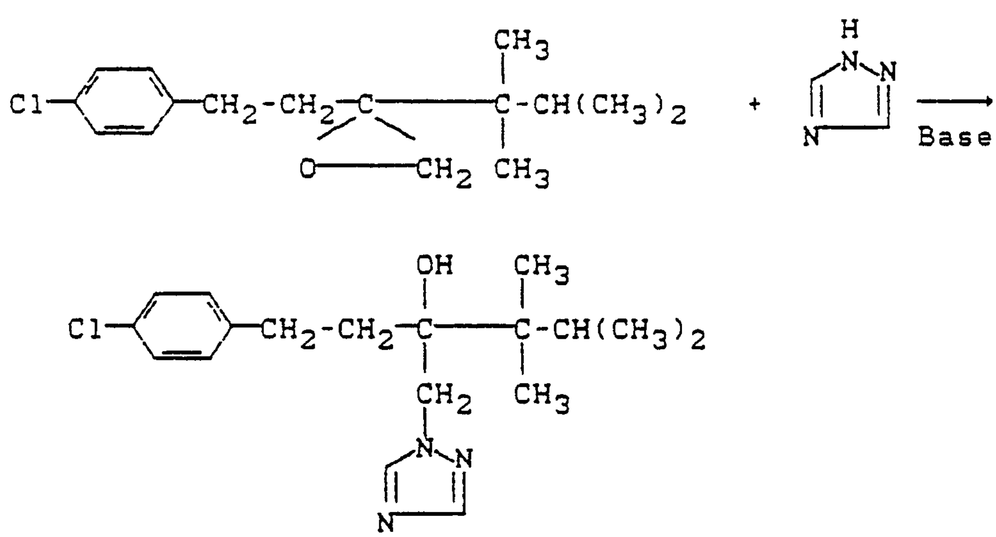

Die bei der Durchführung des erfindungsgemäßen Verfahrens als Ausgangsstoffe zu verwendenden Oxirane sind durch die Formel (II) allgemein definiert. In dieser Formel haben X, Z und der Index m vorzugsweise die Bedeutungen, die bereits im Zusammenhang mit der Beschreibung der erfindungsgemäßen Stoffe der Formel (I) für diese Substituenten bzw. für den Index m als bevorzugt genannt wurden.

Die Oxirane der Formel (II) sind noch nicht bekannt. Sie lassen sich herstellen, indem man

a) in einer ersten Stufe Ketone der Formel

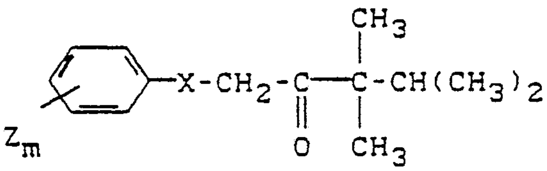

(IV)

in welcher
X, Z und m die oben angegebene Bedeutung haben,
mit Methyl-triphenyl-phosphoniumbromid der Formel

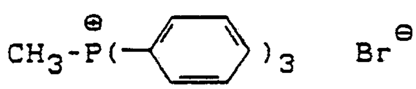

(V)

in Gegenwart einer Base und in Gegenwart eines Verdünnungsmittels umsetzt und

b) in einer zweiten Stufe die so erhaltenen Verbindungen der Formel

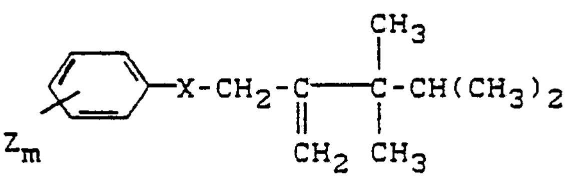

(VI)

in welcher
X, Z und m die oben angegebene Bedeutung haben,
mit Persäuren in Gegenwart eines Verdünnungsmittels umsetzt.

Die bei der Herstellung der Oxirane der Formel (II) als Ausgangsstoffe benötigten Ketone der Formel (IV) sind bekannt oder lassen sich nach im Prinzip bekannten Verfahren in einfacher Weise herstellen (vgl. EP-OS 0 084 834). So erhält man diejenigen Ketone der Formel (IV), in denen X für $CH_2$ steht, dadurch, daß man das Keton der Formel

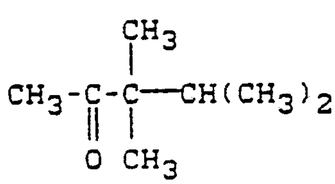

(VII)

mit Aldehyden der Formel

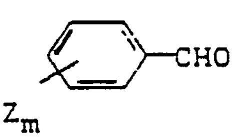

(VIII)

in welcher
Z und m die oben angegebene Bedeutung haben,
in Gegenwart eines Verdünnungsmittels, wie z.B. Ethanol, bei Temperaturen zwischen 0 und 80 °C umsetzt

und die dabei entstehenden Verbindungen der Formel

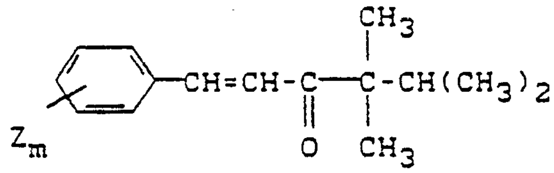

(IX)

in welcher

Z und m die oben angegebene Bedeutung haben,

mit Wasserstoff in Gegenwart eines Katalysators, wie z.B. Raney-Nickel, in Gegenwart eines Verdünnungsmittels, wie z.B. Toluol, bei Temperaturen zwischen 40 und 180 °C hydriert.

Ferner lassen sich diejenigen Ketone der Formel (IV), in denen X für Sauerstoff oder Schwefel steht, herstellen, indem man Halogenketone der Formel

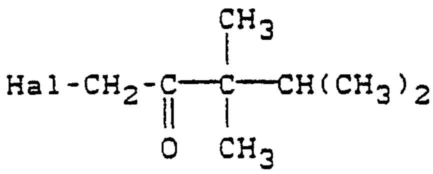

(X)

in welcher

Hal für Chlor oder Brom steht,

mit Phenolen oder Thiophenolen der Formel

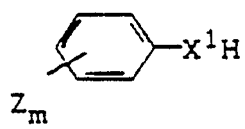

(XI)

in welcher

$X^1$ für Sauerstoff oder Schwefel steht und

Z und m die oben angegebene Bedeutung haben,

in Gegenwart, einer Base und in Gegenwart eines Verdünnungsmittels, wie z.B. Aceton, bei Temperaturen zwischen 10 und 120 °C umsetzt.

Das bei der Herstellung der Oxirane der Formel (II) weiterhin als Ausgangsmaterial benötigte Methyl-triphenyl-phosphonium-bromid der Formel (V) ist bekannt.

Die bei der Herstellung der Oxirane der Formel (II) nach dem obigen Verfahren in der zweiten Stufe als Ausgangssubstanzen benötigten Verbindungen der Formel (VI) sind bisher noch nicht bekannt.

Bei dem Verfahren zur Herstellung der Oxirane der Formel (II) arbeitet man in der ersten Stufe in Gegenwart einer Base. Dabei kommen als Basen alle üblicherweise für Wittig-Reaktionen dieser Art verwendbaren Basen in Betracht. Vorzugsweise verwendbar ist Kalium-tert.-butylat.

Als Verdünnungsmittel kommen bei der Durchführung der ersten Stufe des obigen Verfahrens zur Herstellung der Oxirane der Formel (II) alle für derartige Umsetzungen üblichen organischen Solventien infrage. Vorzugsweise verwendbar sind aromatische Kohlenwasserstoffe, wie Benzol, Toluol und Xylol.

Bei der Durchführung der zweiten Stufe des obigen Verfahrens zur Herstellung der Oxirane der Formel (II) kommen als Reagenzien zur Epoxidierung alle üblichen Persäuren in Betracht. Vorzugsweise verwendbar sind meta-Chlorperbenzoesäure und Peressigsäure. Ferner ist es auch möglich, ein Gemisch aus Essigsäure und Wasserstoffperoxid einzusetzen.

Als Verdünnungsmittel kommen bei der Durchführung der zweiten Stufe des obigen Verfahrens zur Herstellung der Oxirane der Formel (II) alle für derartige Epoxidierungen üblichen Solventien infrage. Vorzugsweise verwendbar sind Dichlormethan, Toluol und Chlorbenzol.

Die Reaktionstemperaturen können bei der Durchführung des Verfahrens zur Herstellung der Oxirane der Formel (II) innerhalb eines bestimmten Bereiches variiert werden. Im allgemeinen arbeitet man in der ersten Stufe bei Temperaturen zwischen 50 °C und 140 °C, vorzugsweise zwischen 80 °C und 120 °C. In

der zweiten Stufe arbeitet man im allgemeinen zwischen 10 °C und 60 °C, vorzugsweise zwischen 20 °C und 50 °C.

Bei der Durchführung des Verfahrens zur Herstellung der Oxirane der Formel (II) geht man im allgemeinen so vor, daß man in der ersten Stufe auf 1 Mol an Keton der Formel (IV) zwischen 1 und 3 Mol Methyl-triphenylphosphonium-bromid der Formel (V) sowie zwischen 1 und 3 Mol Base einsetzt. In der zweiten Stufe setzt man auf 1 Mol an einer Verbindung der Formel (VI) jeweils zwischen 1 und 2 Mol an Persäure ein. Die Aufarbeitung erfolgt jeweils nach üblichen Methoden.

Diejenigen Oxirane der Formel (II), in denen X für Sauerstoff oder Schwefel steht, lassen sich auch herstellen, indem man

c) Ketone der Formel

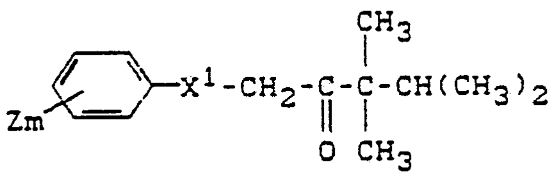

(IVa)

in welcher

Z und m die oben angegebene Bedeutung haben und

$X^1$ für Sauerstoff oder Schwefel steht,

entweder

α) mit Dimethyloxosulfonium-methylid der Formel

$$(CH_3)_2\overset{\delta\oplus}{S}O\overset{\delta\ominus}{C}H_2 \qquad (XII)$$

oder

β) mit Dimethylsulfonium-methylid der Formel

$$(CH_3)_2\overset{\delta\oplus}{S}\ \ \overset{\delta\ominus}{C}H_2 \qquad (XIII)$$

in Gegenwart eines Verdünnungsmittels umsetzt.

Das bei dem Verfahren (c) als Reaktionskomponente benötigte Dimethyloxosulfonium-methylid der Formel (XII) ist bekannt (vgl. J. Amer. Chem. Soc. 87, 1363 (1965)). Es wird bei der obigen Umsetzung in frisch zubereitetem Zustand verarbeitet, indem man es in situ durch Umsetzung von Trimethyl-oxo-sulfonium-iodid mit Natriumhydrid oder Natriumamid, insbesondere mit Kalium-tert.-butylat oder Natriummethylat, in Gegenwart eines Verdünnungsmittels erzeugt.

Das bei dem Verfahren (c) außerdem als Reaktionskomponente in Betracht kommende Dimethylsulfonium-methylid der Formel (XIII) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfonium-halogenid oder Trimethylsufonium-methylsulfat, in Gegenwart einer starken Base, wie zum Beispiel Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (c) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei dem Verfahren (c) in einem größeren Bereich variiert werden. Im allgemeinen arbeitet man bei Temperaturen zwischen 0 und 100 °C, vorzugsweise zwischen 10 und 60 °C.

Bei der Durchführung des Verfahrens (c) man auf 1 Mol an Keton der Formel (IVa) vorzugsweise 1 bis 3 Mol an Dimethyloxosulfonium-methylid der Formel (XII) bzw. an Dimethylsulfonium-methylid der Formel (XIII) ein. Die Isolierung der Oxirane erfolgt nach üblichen Methoden.

Die Oxirane der Formel (II) können bei dem erfindungsgemäßen Verfahren gegebenenfalls ohne Isolierung direkt weiter umgesetzt werden.

Als Verdünnungsmittel kommen für das erfindungsgemäße Verfahren unter den Reaktionsbedingungen inerte organische Lösungsmittel infrage. Vorzugsweise verwendbar sind Alkohole, wie z.B. Ethanol, Methoxyethanol oder Propanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid. Es kann auch von Vorteil sein, eine geringe Menge Wasser hinzuzufügen.

Als Basen kommen für das erfindungsgemäße Verfahren alle üblicherweise verwendbaren anorganischen und organischen Basen in Betracht. Vorzugsweise verwendbar sind Alkalimetallcarbonate, wie z.B. Natrium- und Kaliumcarbonat; Alkalimetallhydroxide, wie z.B. Natriumhydroxid; Alkalimetallalkoholate, wie z.B. Natrium- und Kaliummethylat und -ethylat; Alkalimetallhydride, wie z.B. Natriumhydrid; sowie niedere tertiäre Alkylamine, Cycloalkylamine und Aralkylamine, wie insbesondere Triethylamin.

Als Katalysatoren können bei der Durchführung des erfindungsgemäßen Verfahrens alle für derartige Umsetzungen üblichen Reaktionsbeschleuniger eingesetzt werden. Vorzugsweise verwendbar ist $\alpha,\alpha'$-Azoisobutyronitril.

Die Reaktionstemperaturen können bei der Durchführung des erfindungsgemäßen Verfahrens in einem größeren Bereich variiert werden. Im allgemeinen arbeitet man bei Temperaturen zwischen 0 und 200 °C, vorzugsweise zwischen 60 und 150 °C.

Bei der Durchführung des erfindungsgemäßen Verfahrens arbeitet man im allgemeinen unter Normaldruck. Es ist jedoch auch möglich, unter erhöhtem oder vermindertem Druck zu arbeiten.

Bei der Durchführung des erfindungsgemäßen Verfahrens setzt man auf 1 Mol Oxiran der Formel (II) vorzugsweise 1 bis 2 Mol an 1,2,4-Triazol und gegebenenfalls 1 bis 2 Mol an Säurebindemittel ein. Die Aufarbeitung und die Isolierung der Endprodukte erfolgt nach üblichen Methoden.

Die nach dem erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen infrage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seinen einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;

(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Mit besonders gutem Erfolg lassen sich die erfindungsgemäßen Stoffe zur Bekämpfung von Reiskrankheiten, wie Pellicularia sasakii und Pyricularia oryzae, einsetzen. Außerdem eignen sie sich sehr gut zur Bekämpfung von Puccinia recondita, Cochliobolus sativus und Leptosphaeria nodorum.
Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was

wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in Polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmi gen Strechmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als

feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus orgnaischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsgulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungemäßen Stoffe geht aus den folgenden Beispielen hervor.

Herstellungsbeispiele

Beispiel 1

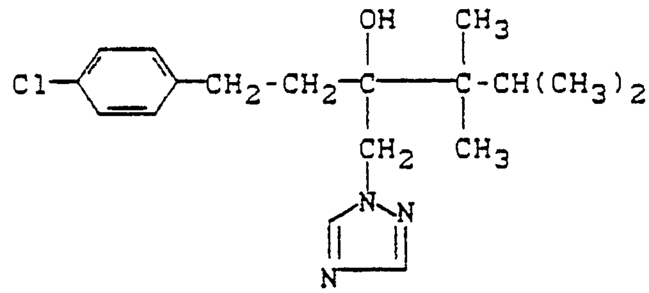

(I-1)

Eine Lösung von 14,0 g (0,052 Mol) 2-(4-Chlorphenylethyl)-2-(1,1,2-trimethyl-propyl)-oxiran, 6,9 g (0,1 Mol) 1,2,4-Triazol, 0,4 g Natriumhydroxid, 0,4 ml Wasser und einer Spatelspitze α,α'-Azo-isobutyro-nitril in 60 ml Dimethylformamid wird 10 Stunden auf 120° C erwärmt. Danach kühlt man auf Raumtemperatur ab, engt durch Abzeihen des Lösungsmittels unter vermindertem Druck ein, löst den verbleibenden Rückstand in Dichlormethan, wäscht dreimal mit Wasser, trocknet die organische Phase über Natriumsulfat und entfernt das Lösungsmittel unter vermindertem Druck. Der Rückstand wird durch Säulenchromatographie (Kieselgel; Dichlormethan:Essigester = 1:1) gereinigt. Man erhält auf diese Weise 8,1 g (46% der Theorie) an 1-(4-Chorphenyl)-3-(1,2,4-triazol-1-yl-methyl)-4,4,5-trimethyl-hexan-3-ol in Form einer Festsubstanz, die nach Umkristallisation aus Diisopropylether einen Schmelzpunkt von 76 - 78° C aufweist.

Herstellung von Ausgangsprodukten

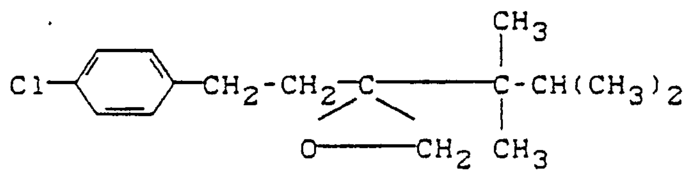

(II-1)

In eine siedende Lösung von 14,5 g (0,0579 Mol) 2-(4-Chlorphenylethyl)-3,3,4-trimethyl-pent-1-en in 60 ml Dichlormethan wird innerhalb von 1,5 Stunden eine Lösung von 11,7 g (0,068 Mol) m-Chlorperbenzoesäure in 150 ml Dichlormethan eingetropft. Man erhitzt weitere 4 Stunden unter Rückfluß, kühlt dann auf Raumtemperatur ab, wäscht zunächst dreimal mit 1N wäßriger Natronlauge und danach mit Wasser, trocknet dann die organische Phase über Natriumsulfat und engt durch Abziehen des Lösungsmittels unter vermindertem Druck ein. Man erhält 14,0 g eines gelben Öles, das gemäß gaschromatographischer und massenspektrometrischer Analyse zu 88 % aus 2-(4-Chlorphenylethyl)-2-(1,1,2-trimethyl-propyl)-oxiran besteht. Die Ausbeute errechnet sich danach zu 80 % der Theorie. Das Produkt wird ohne zusätzliche Reinigung für die weitere Umsetzung verwendet.

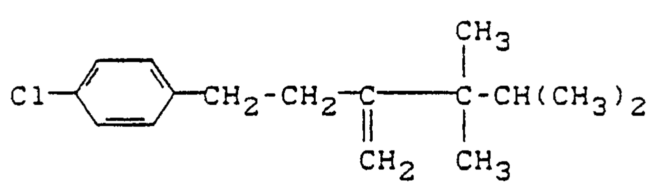

(VI-1)

Eine Suspension aus 47,4 g (0,133 Mol) Methyltriphenylphosphonium-bromid und 15,3 g (0,137 Mol) Kalium-tert.-butylat in 250 ml absolutem Toluol wird unter trockenem Stickstoff unter Rückfluß erhitzt. Innerhalb von 5 Minuten werden 25,2 g (0,1 Mol) 1-(4-Chlorphenyl)-4,4,5-trimethyl-hexan-3-on eingetragen. Das Reaktionsgemisch wird weitere 15 Stunden unter Rückfluß erhitzt, dann auf Raumtemperatur abgekühlt, zweimal mit Wasser gewaschen und unter vermindertem Druck eingeengt. Man nimmt den Rückstand in Essigester auf, kühlt auf 5° C ab und saugt den gebildeten Kristallbrei ab. Das Filtrat wird eingeengt und

unter vermindertem Druck destilliert. Man erhält 14,5 g (58 % der Theorie) an 2-(4-Chlorphenylethyl)-3,3,4-trimethyl-pent-1-en in Form eines gelben Öles von Siedepunkt 88-90° C/0,1 mbar.

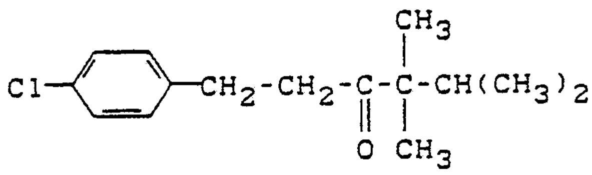

(IV-1)

Eine Lösung von 33,1 g (0,132 Mol) 1-(4-Chlorphenyl)-4,4,5-trimethyl-1-hexen-3-on in 200 ml Toluol wird mit 5 g Raney-Nickel versetzt und im Autoklaven 5 Stunden bei 80° C unter einem Wasserstoffdruck von 80 - 100 bar gerührt. Danach wird das Reaktionsgemisch filtriert und unter vermindertem Druck eingeengt. Man erhält 28,3 g (85 % der Theorie) an 1-(4-Chlorphenyl)-4,4,5-trimethylhexan-3-on in Form eines gelben Öles.

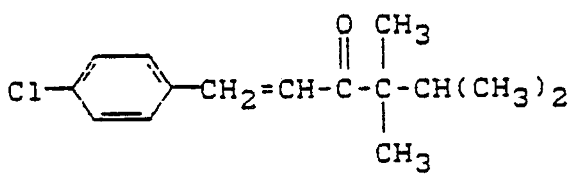

(IX-1)

44,4 g (0,316 Mol) 4-Chlorbenzaldehyd und 40,5 g (0,316 Mol) 3,3,4-Trimethyl-pentan-2-on werden in einem Gemisch aus 100 ml Ethanol und 10 ml Wasser gelöst und mit einer Lösung von 0,9 g Natriumhydroxid in 10 ml Wasser versetzt. Das Gemisch wird zunächst 1 Stunde bei Raumtemperatur gerührt, dann mit 0,4 g festem Natriumhydroxid versetzt und weitere 16 Stunden gerührt. Der entstandene Niederschlag wird abgesaugt und mit Wasser nachgewaschen. Man erhält 33,1 g (42 % der Theorie) an 1-(4-Chlorphenyl)-4,4,5-trimethyl-1-hexan-3-on in Form eines niedrig schmelzenden, ölig-kristallinen Produktes.

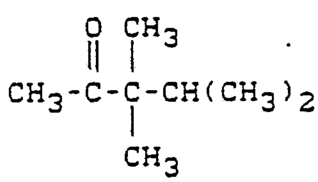

(VII-1)

Zu einer Lösung von 209 g (1,2 Mol) Natriumdithionit und 540 g (6 Mol) Natriumhydrogencarbonat in einem Gemisch aus 1 l Dimethylformamid und 1 l Wasser werden 97,2 g (0,6 Mol) 1-Chlor-3,3,4-trimethyl-pentan-2-on gegeben. Das Gemisch wird 1 Stunde bei 70° C gerührt. Danach filtriert man das Reaktionsgemisch, wäscht den Feststoff mit Ether und extrahiert das Filtrat zweimal mit Ether.

Die vereinigten Etherphasen werden zweimal mit Wasser gewaschen und nach dem Trocknen über Natriumsulfat unter vermindertem Druck eingeengt. Man erhält 42 g einer farblosen Flüssigkeit, die gemäß gaschromatographischer Analyse zu über 90 % aus 3,3,4-Trimethyl-pentan-2-on besteht. Die Ausbeute errechnet sich danach zu 49 % der Theorie.

Beispiel 2

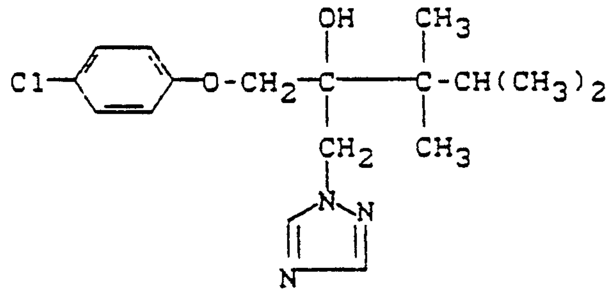

(I-2)

Zu einer siedenden Lösung von 13,4 g (0,14 Mol) Natriumbutanolat und 97 g (1,4 Mol) 1,2,4-Triazol in 400 ml n-Butanol werden in 1,5 Stunden 376 g rohes 2-(4-Chlorphenoxymethyl)-2-(1,1,2-trimethylpropyl)-oxiran gelöst in 400 ml n-Butanol zugetropft. Nach 22-stündigem Kochen unter Rückfluß wird das Lösungsmittel unter vermindertem Druck abdestilliert und der Rückstand mit 500 ml Dichlormethan versetzt. Man filtriert von ausgeschiedenen 2-(4-Chlorphenoxymethyl)-1-(1,2,4-triazol-4-yl)-3,3,4-trimethylpentan-2-ol (15 g; Schmelzpunkt 173-174° C) ab, wäscht das Filtrat zweimal mit je 250 ml Wasser, trocknet über wasserfreiem Natriumsulfat und destilliert das Lösungsmittel ab. Der Rückstand wird über Kieselgel mit Trichlormethan als Fließmittel chromatographiert. Man erhält so 174 g 2-(4-Chlorphenoxymethyl)-1-(1,2,4-triazol-1-yl)-3,3,4-trimethylpentan-2-ol in Form farbloser Kristalle vom Schmelzpunkt 59-60° C.

Herstellung von Ausgangsprodukten

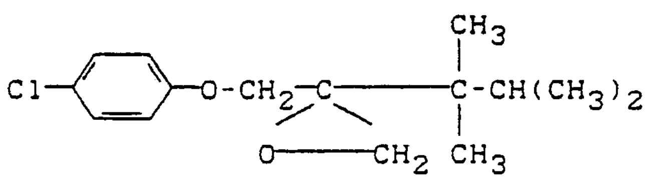

(II-2)

Zu einer Lösung von 220 ml (3 Mol) Dimethylsulfid in 488 ml tert.-Butanol werden unter Außenkühlung bei 20-30° C 285 ml (3 Mol) Dimethylsulfat in 1,5 Stunden zugetropft. Man läßt das Gemisch bei 20-25° C noch 3 Stunden rühren, gibt dann 382,2 g (1,5 Mol) 1-(4-Chlorphenoxy)-3,3,4-Trimethylpentan-2-on gelöst in 380 ml tert.-Butanol zu und trägt anschließend unter Außenkühlung bei 20-30° C 336 g (6 Mol) gepulvertes Kaliumhydroxid im Verlaufe von 1 Stunde ein. Nach 16 Stunden werden 375 ml Toluol und 375 ml Wasser sowie 187 ml Chlorlauge zugegeben. Die organische Phase wird abgetrennt, dreimal mit je 1000 ml Wasser gewaschen, über wasserfreiem Natriumsulfat getrocknet und unter vermindertm Druck eingedampft. Das so erhaltene rohe 2-(4-Chlorphenoxymethyl) -2- (1,1,2-trimethylpropyl)-oxiran (393 g) wird ohne weitere Reinigung weiter umgesetzt.

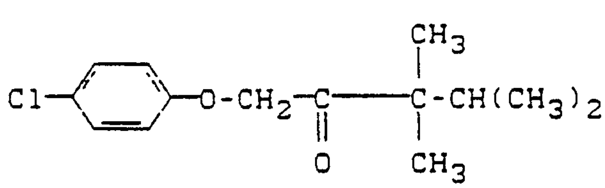

(IV-2)

Zu einem auf 100° C erhitzten Gemisch aus 364,9 g (2,84 Mol) 4-Chlorphenol und 392 g (2,84 Mol) Kaliumcarbonat in 2700 ml N,N-Dimethylformamid werden innerhalb 1 Stunde 461,5 g (2,84 Mol) 1-Chlor-3,3,4-trimethylpentan-2-on unter Rühren zugegeben. Nach zehnstündigem Erhitzen auf 100° C wird auf 20° C gekühlt, vom ausgeschiedenen Kaliumchlorid abfiltriert und das Filtrat unter vermindertem Druck eingedampft. Der Rückstand wird in 1500 ml Dichlormethan aufgenommen und zweimal mit je 750 ml 5 %iger wäßriger Natronlauge und 750 ml Wasser gewaschen. Nach dem Trocknen der organischen Phase über wasserfreiem Natriumsulfat wird das Lösungsmittel unter vermindertem Druck abdestilliert und der

ölige Rückstand im Vakuum destilliert. Man erhält so 632 g (87,4 % der Theorie) 1-(4-Chlorphenoxy)-3,3,4-trimethylpentan-2-on vom Siedepunkt 147-148° C/0,15 Torr.

Nach der im Beispiel 2 angegebenen Methode werden auch die im folgenden Beispiel aufgeführten Stoffe hergestellt:

Beispiel 3

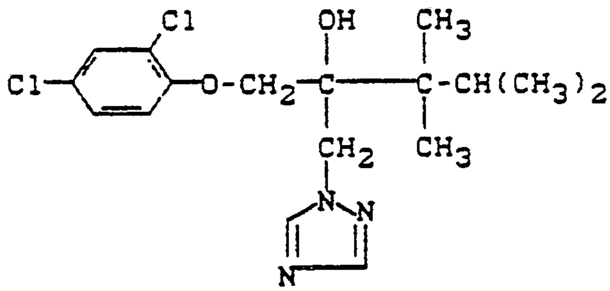

(I-3)

$n_D^{25}$ = 1,5363

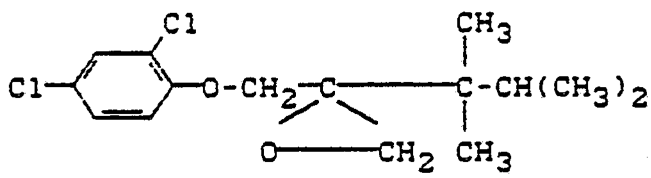

(II-3)

Das Oxiran wird ohne zusätzliche Reinigung direkt weiter umgesetzt.

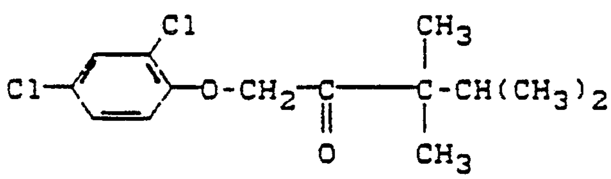

(IV-3)

Das Keton wird ohne weitere Reinigung als Rohprodukt weiter umgesetzt.

Beispiel 4

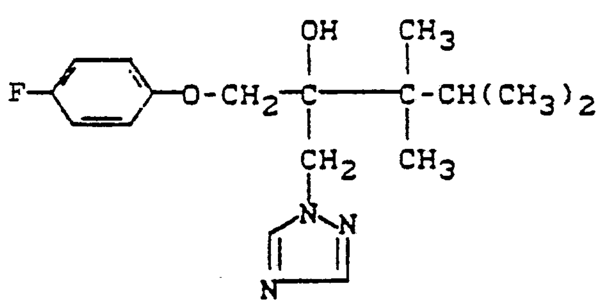

(I-4)

Öl

In den folgenden Verwendungsbeispielen wurden die nachstehend aufgeführten Verbindungen als Vergleichssubstanzen eingesetzt:

(A) = 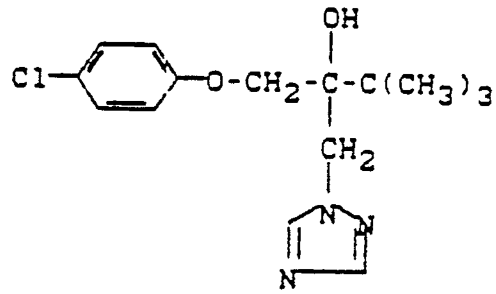

(B) = 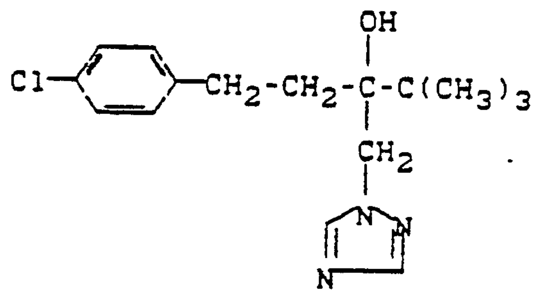

Beispiel A

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20° C und 100 % relativer Luftfeuchtigkeit in einer Inkubationskabine.

Die Pflanzen werden dann im Gewächshaus bei 20° C und einer relativen Luftfeuchtigkeit von ca. 70 % aufgestellt.

12 Tage nach der Inokulation erfolgt die Auswertung.

In diesem Test zeigt die erfindungsgemäße Verbindung der Formel (I-1) eine sehr gute Wirkung.

Beispiel B

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24° C und bei einer relativen Luftfeuchtigkeit von ca. 75 % im Gewächshaus aufgestellt.

10 Tage nach der Inokulation erfolgt die Auswertung.

In diesem Test zeigt die erfindungsgemäße Verbindung der Formel (I-2) eine bessere Wirkung als die Vergleichssubstanz (A).

Beispiel C

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung der Formel (I-1) eine sehr gute Wirkung.

Beispiel D

Puccinia-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung mit protektive Wirksamkeit werden junge Pflanzen mit einer Sporensuspension von Puccinia recondita mit in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20° C und 100 % rel. Luftfeuchtigkeit in einer Inkubationskabine.

Die Pflanzen werden in einem Gewächshaus bei einer Temperatur von ca. 20° C und einer relativen Luftfeuchtigkeit von ca. 80 % aufgestellt, um die Entwicklung von Rostpusteln zu begünstigen.

Die Auswertung erfolgt 10 Tage nach der Inokulation.

In diesem Test zeigt die erfindungsgemäße Verbindung der Formel (I-1) eine sehr gute Wirkung.

Beispiel E

Cochliobolus sativus-Test (Gerste) / protektiv

Lösungsmittel: 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20° C und 100 % rel. Luftfeuchtigkeit in einer Inkubationskabine.

Die Pflanzen werden in einem Gewächshaus bei einer Temperatur von ca. 20° C und einer relativen Luftfeuchtigkeit von ca. 80 % aufgestellt.

7 Tage nach der Inokulation erfolgt die Auswertung.

In diesem Test zeigt die erfindungsgemäße Verbindung der Formel (I-I) eine sehr gute Wirkung.

Beispiel F

Leptosphaeria nodorum-Test (Weizen) / protektiv

Lösungsmittel: 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20° C und 100 % rel. Luftfeuchtigkeit in einer Inkubationskabine.

Die Pflanzen werden in einem Gewächshaus bei einer Temperatur von ca. 15° C und einer relativen Luftfeuchtigkeit von ca. 80 % aufgestellt.

10 Tage nach der Inokulation erfolgt die Auswertung.

In diesem Test zeigen die erfindungsgemäßen Verbindungen der Formeln (I-1) und (I-2) eine bessere Wirkung als die Vergleichssubstanzen (A) und (B).

Beispiel G

Pyrenophora teres-Test (Gerste) / protektiv

Lösungsmittel: 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20° C und 100 % rel. Luftfeuchtigkeit in einer Inkubationskabine.

Die Pflanzen werden in einem Gewächshaus bei einer Temperatur von ca. 20° C und einer relativen Luftfeuchtigkeit von ca. 80 % aufgestellt.

7 Tage nach der Inokulation erfolgt die Auswertung.

In diesem Test zeigt die erfindungsgemäße Verbindung der Formel (I-1) eine sehr gute Wirkung.

Beispiel H

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25° C und 100 % relativer Luftfeuchtigkeit aufgestellt.

5 bis 8 Tage nach der Inokulation erfolgt die Auswertung des Krankheitsbefalles.

In diesem Test zeigt die erfindungsgemäße Verbindung der Formel (I-1) eine sehr gute Wirkung.

Beispiel I

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalls.

In diesem Test zeigt die erfindungsgemäße Verbindung der Formel (I-1) eine bessere Wirkung als die Vergleichssubstanz (B).

Beispiel K

Pflanzenverträglichkeitstest

Testpflanze: Gurke
Versuchsdauer: 7 Tage
Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Mit dieser Wirkstoffzubereitung bespritzt man junge Pflanzen bis zur Tropfnässe und stellt sie in einem

Gewächshaus bei ca. 20° C auf.

Die Pflanzen werden auf Schäden wie Wuchsbeeinträchtigungen, Verfärbungen und Nekrosen ausgewertet. Angegeben wird der Schädigungsgrad der Pflanzen in %. Dabei bedeutet:

0 %: keine Schäden

100 %: Pflanze vollkommen geschädigt.

In diesem Test zeigen die erfindungsgemäßen Verbindungen der Formeln (I-1) und (I-2) eine bessere Verträglichkeit als die Vergleichssubstanzen (A) und (B).

## Ansprüche

1. Hydroxyalkyl-azolyl-Derivate der Formel

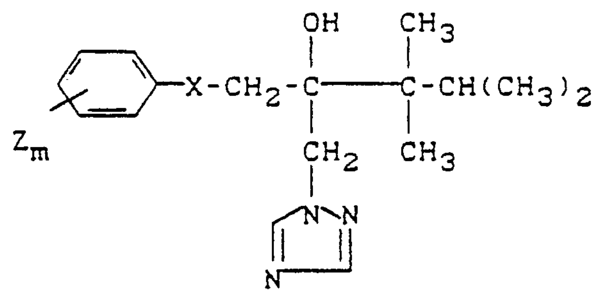

(I)

in welcher

Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl oder Alkoximinomethyl mit 1 bis 4 Kohlenstoffatomen in der Alkoxygruppe steht,

X für Sauerstoff, Schwefel oder eine $CH_2$-Gruppe steht und

m für die Zahlen 0, 1, 2 oder 3 steht,

sowie deren Säureadditions-Salze und Metallsalz-Komplexe.

2. Hydroxyalkyl-azolyl-Derivate der Formel (I) gemäß Anspruch 1, in denen

X für Sauerstoff, Schwefel oder eine $CH_2$-Gruppe steht,

Z für Fluor, Chlor, Brom, Methyl, Ethyl, Trifluormethyl, Trifluormethoxy, Trifluormethylthio, Phenyl, Methoximinomethyl oder Ethoximinomethyl steht und

m für die Zahlen 0, 1, 2 oder 3 steht.

3. Verfahren zur Herstellung von Hydroxyalkyl-azolyl-Derivaten der Formel

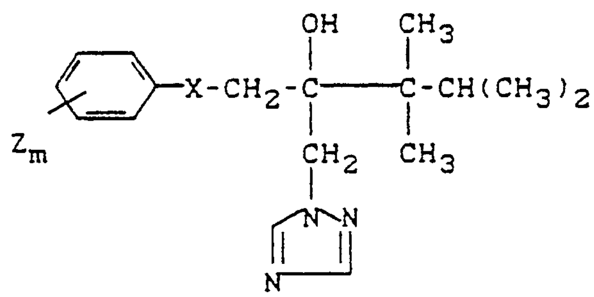

(I)

in welcher

Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogen atomen, Phenyl oder Alkoximinomethyl mit 1 bis 4 Kohlenstoffatomen in der Alkoxygruppe steht,

X für Sauerstoff, Schwefel oder eine $CH_2$-Gruppe steht und

m für die Zahlen 0, 1, 2 oder 3 steht,

sowie von deren Säureadditions-Salzen und Metallsalz-Komplexen, dadurch gekennzeichnet, daß man Oxirane der Formel

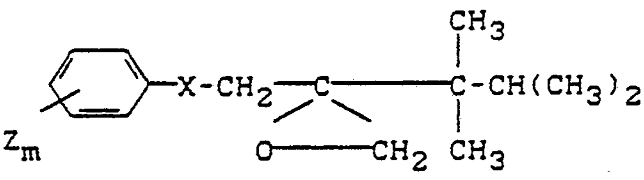

(II)

in welcher
X, Z und m die oben angegebene Bedeutung haben,
mit 1,2,4-Triazol der Formel

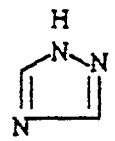

(III)

in Gegenwart eines Verdünnungsmittels und gegebenenfalls in Gegenwart eines Säurebindemittels sowie gegebenenfalls in Gegenwart eines Katalysators umsetzt und gegebenenfalls anschließend an die so erhaltenen Verbindungen der Formel (I) eine Säure oder ein Metallsalz addiert.

4. Fungizide und pflanzenwuchsregulierende Mittel, gekennzeichnet durch einen Gehalt an mindestens einem Hydroxyalkyl-azolyl-Derivat der Formel (I) gemäß Anspruch 1 bzw. an einem Säureadditions-Salz oder Metallsalz-Komplex eines Hydroxyalkyl-azolyl-Derivates der Formel (I).

5. Verfahren zur Bekämpfung von Pilzen und zur Regulierung des Pflanzenwachstums, dadurch gekennzeichnet, daß man Hydroxyalkyl-azolyl-Derivate der Formel (I) gemäß Anspruch 1 bzw. deren Säureadditions-Salze oder Metallsalz-Komplexe auf die Pflanzen und/oder deren Lebensraum ausbringt.

6. Verwendung von Hydroxyalkyl-azolyl-Derivaten der Formel (I) gemäß Anspruch 1 bzw. von deren Säureadditions-Salzen und Metallsalz-Komplexen zur Bekämpfung von Pilzen sowie zur Regulierung des Pflanzenwachstums.

7. Verfahren zur Herstellung von fungiziden und pflanzenwuchsregulierenden Mitteln, dadurch gekennzeichnet, daß man Hydroxyalkyl-azolyl-Derivate der Formel (I) gemäß Anspruch 1 bzw. deren Säureadditionssalze oder Metallsalz-Komplexe mit Streckmitteln und/oder oberflächenaktiven Stoffen vermischt.

8. Oxirane der Formel

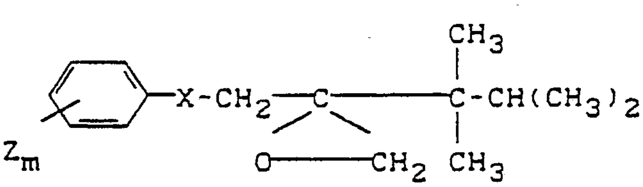

(II)

in welcher
Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl oder Alkoximinomethyl mit 1 bis 4 Kohlenstoffatomen in der Alkoxygruppe steht,
X für Sauerstoff, Schwefel oder eine $CH_2$-Gruppe steht und
m für die Zahlen 0, 1, 2 oder 3 steht.

9. Verfahren zur Herstellung von Oxiranen der Formel

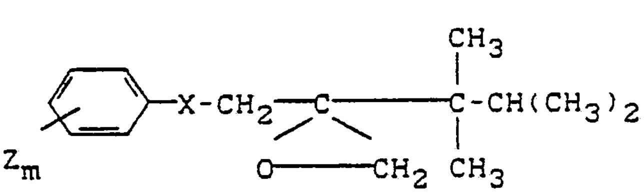

(II)

in welcher
Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl oder Alkoximinomethyl mit 1 bis 4 Kohlenstoffatomen in der Alkoxygruppe steht,
X für Sauerstoff, Schwefel oder eine $CH_2$-Gruppe steht und
m für die Zahlen 0, 1, 2 oder 3 steht,
dadurch gekennzeichnet, daß man

a) in einer ersten Stufe Ketone der Formel

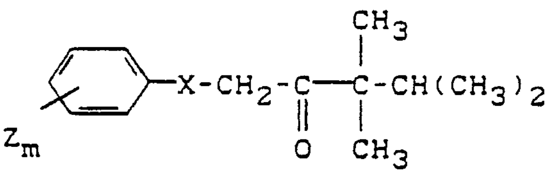

(IV)

in welcher
X, Z und m die oben angegebene Bedeutung haben,
mit Methyl-triphenyl-phosphoniumbromid der Formel

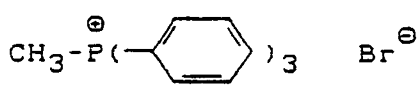

(V)

in Gegenwart einer Base und in Gegenwart eines Verdünnungsmittels umsetzt und

b) in einer zweiten Stufe die so erhaltenen Verbindungen der Formel

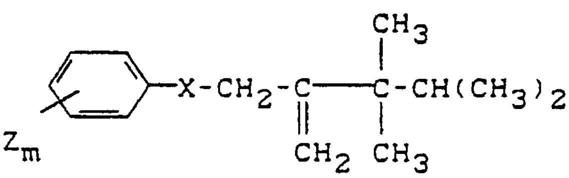

(VI)

in welcher
X, Z und m die oben angegebene Bedeutung haben,
mit Persäuren in Gegenwart eines Verdünnungsmittels umsetzt,
oder indem man

c) Ketone der Formel

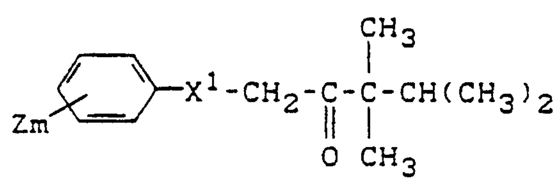

(IVa)

in welcher
Z und m die oben angegebene Bedeutung haben und
$X^1$ für Sauerstoff oder Schwefel steht,
entweder
α) mit Dimethyloxosulfonium-methylid der Formel

$$\overset{\delta\oplus\,\delta\ominus}{(CH_3)_2SOCH_2} \qquad (XII)$$

oder
β) mit Dimethylsulfonium-methylid der Formel

$$\overset{\delta\oplus}{(CH_3)_2S}\ \ \overset{\delta\ominus}{CH_2} \qquad (XIII)$$

in Gegenwart eines Verdünnungsmittels umsetzt.

10. Verbindungen der Formel

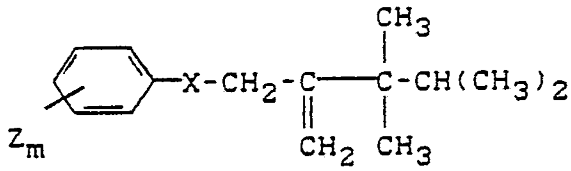

(VI)

in welcher
Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl oder Alkoximinomethyl mit 1 bis 4 Kohlenstoffatomen in der Alkoxygruppe steht,
X für Sauerstoff, Schwefel oder eine $CH_2$-Gruppe steht und
m für die Zahlen 0, 1, 2 oder 3 steht.